# EUROPEAN PATENT SPECIFICATION

(11) **EP 3 839 485 B1**
(45) Date of publication and mention of the grant of the patent: **13.03.2024**
(21) Application number: 19850656.0
(22) Date of filing: 13.08.2019
(51) Int. Cl.: G01N 21/76, G01N 21/63, G01N 33/543

(54) **MICROSPHERE COMPOSITION FOR CHEMILUMINESCENCE ANALYSIS AND USE THEREOF**
MIKROKUGELZUSAMMENSETZUNG FÜR CHEMILUMINESZENZANALYSE UND DEREN VERWENDUNG
COMPOSITION DE MICROSPHÈRES POUR ANALYSE PAR CHIMILUMINESCENCE ET SON UTILISATION

(30) Priority: 13.08.2018 CN 201810915144
(43) Date of publication of application: 23.06.2021
(73) Proprietor: Chemclin Diagnostics (Shanghai) Co., Ltd., Pudong, Shanghai 201210 (CN); Chemclin Diagnostics Co., Ltd., Beijing 100094 (CN)
(72) Inventor: YANG, Yang, Shanghai 201210 (CN); KANG, Caijun, Shanghai 201210 (CN); ZHAO, Weiguo, Shanghai 201210 (CN); LIU, Yuhui, Beijing 100094 (CN); LI, Lin, Beijing 100094 (CN)
(74) Representative: Cabinet Laurent & Charras
(86) International application number: PCT/CN2019/100340
(87) International publication number: WO 2020/034938

(56) References cited:
- WO-A1-03/042699
- CN-A- 102 575 156
- CN-A- 103 134 926
- CN-A- 103 743 722
- CN-A- 106 267 216
- CN-A- 108 169 497
- US-A1- 2009 029 347
- Bangs Laboratories: "TechNote 301, Immunological application, BEADS ABOVE THE REST", Immunological Application 9025 Technology Dr. @BULLET Fishers, IN 46038-2886 800 Superparamagnetic Microsphere Based Assays IX. Proximity Assays A. Scintillation Proximity Assay B. Luminescent Oxygen Channeling Immunoassay C. Third Wave Assay X. Micr, 20 March 2013 (2013-03-20), pages 1-13, XP055905326, Retrieved from the Internet: URL:https://www.bangslabs.com/sites/defaul t/files/imce/docs/TechNote%20301%20Web.pdf [retrieved on 2022-03-25]
- LINDMO T ET AL: "Immunometric assay by flow cytometry using mixtures of two particle types of different affinity", JOURNAL OF IMMUNOLOGICAL METHODS, ELSEVIER SCIENCE PUBLISHERS B.V.,AMSTERDAM, NL, vol. 126, no. 2, 9 February 1990 (1990-02-09), pages 183-189, XP023973551, ISSN: 0022-1759, DOI: 10.1016/0022-1759(90)90149-P [retrieved on 1990-02-09]
- T M Mchugh ET AL: "Simultaneous detection of antibodies to cytomegalovirus and herpes simplex virus by using flow cytometry and a microsphere-based fluorescence immunoassay", Journal of Clinical Microbiology, 1 October 1988 (1988-10-01), pages 1957-1961, XP055181994, UNITED STATES Retrieved from the Internet: URL:http://jcm.asm.org/content/26/10/1957. abstract
- TAK FOR YU ZETA ET AL: "Rapid, automated, parallel quantitative immunoassays using highly integrated microfluidics and AlphaLISA", SCIENTIFIC REPORTS, vol. 5, no. 1, 1 September 2015 (2015-09-01), XP055906061, DOI: 10.1038/srep11339 Retrieved from the Internet: URL:https://www.nature.com/articles/srep11 339.pdf>
- LI PENG ET AL: "Establishment of a novel homogeneous nanoparticle-based assay for sensitive procalcitonin detection of ultra low-volume serum samples", INTERNATIONAL JOURNAL OF NANOMEDICINE, vol. Volume 13, 13 September 2018 (2018-09-13), pages 5395-5404, XP055906232, DOI: 10.2147/IJN.S173776 Retrieved from the Internet: URL:https://www.dovepress.com/getfile.php? fileID=44434>

## Description

The present disclosure relates to the technical field of chemiluminescent analysis, and in particular, to a microsphere composition for chemiluminescent analysis and use thereof.

The chemiluminescent analysis method is a method in which light waves emitted by chemiluminescent substances are used to perform detection. Chemiluminescent substances are used as labels in nucleic acid detection and immunodetection. For example, a certain molecule in a specific binding pair can be combined with a luminescent substance through a variety of ways to form a luminescent microsphere composition. The microsphere composition can react with a substance to be detected (i.e., the other molecule in the specific binding pair) in a sample, and be distributed in the solid phase and the liquid phase, the distribution ratio being related to an amount of the substance to be detected. By measuring an amount of luminescence in the solid phase or the liquid phase, a corresponding concentration of the substance to be detected in the sample can be obtained.

At present, with the advancement of the detection industry, there are more and more demands for ultra-sensitive reagents, and both an extremely high sensitivity and a very wide linear range are required. Existing microsphere compositions cannot meet the above detection conditions.

Therefore, there is an urgent need to develop a microsphere composition for chemiluminescent analysis that can meet both the sensitivity requirement and the linear range requirement.

Document US 2009/029347 ; LINDMO T ET AL, "Immunometric assay by flow cytometry using mixtures of two particle types of different affinity", Journal of immunological methods, Vol 126, No 2, 9 Febr 1990 (XP023973551); T M McHugh et al, "Simultaneous detection of antibodies to cytomegalovirus and herpes simplex virus by using flow cytometry and a microsphere-based fluorescence immunoassay", Journal of clinical microbiology, 1 October 1988, (XP 055181994); all disclose flow cytometry immunodetection, the microspheres used being counting fluorescent microsphere, that are obtained by introducing one or more fluorescent microspheres on the surface or inside the polymer microspheres. The fluorescent microspheres can emit fluorescence when excited by a certain energy. Only ordinary colloidal particles are used in the turbidimetric immunoassay disclosed in the article Bangs Laboratories "TechNote 301, Immunological application, BEADS ABOVE THE REST", Immunological Application 9025, 20 March 2013 (XP055905326). These ordinary colloidal particles do not contain any luminescent substances on the surface or inside the particles, and only particle aggregation is formed after antigen-antibody binding, thereby hindering the size of the light transmittance to affect the optical properties and detection signal. Thus, all these documents disclose common microspheres.
TAK FOR ZU BETA ET AL., "Rapid, automated, parallel quantitative immunoassays using highly integrated microfluidics and AlphaLISA", Scientific Reports, Vol N)5, No. 1, 1 September 2015 (XP 055906061) discloses a microsphere composition for chemiluminescent analysis.

The technical problem to be solved by the invention as claimed in claims 1-9 is to provide a microsphere composition for chemiluminescent analysis with respect to the deficiency in existing technologies. When used for chemiluminescent analysis and detection, the microsphere composition has both an ultrahigh sensitivity and a very wide detection range. Besides, the detection time can be shortened.

For this purpose, the inventions relates to a microsphere composition for chemiluminescent analysis, which comprises at least two kinds of receptor microspheres of different particle diameters, and the receptor microspheres are capable of reacting with active oxygen to produce a detectable chemiluminescent signal.

Preferably, the receptor microsphere comprises two portions, i.e., a luminescent composition and a substrate. The luminescent composition is filled in the substrate and/or is coated on a surface of the substrate.

More preferably, the luminescent composition is capable of reacting with active oxygen to produce a detectable chemiluminescent signal, and includes a chemiluminescent compound and a metal chelate.

The chemiluminescent compound may be selected from an olefin compound, preferably selected from thioxene, a dibutanedione compound, dioxene, enol ether, enamine, 9-alkylene xanthane, 9-alkylene-N-9,10 dihydroacridine, aryl etherene, aryl imidazole, lucigenin and their derivatives, and more preferably selected from thioxene and its derivatives. The olefin compound is a compound capable of reacting with the active oxygen (such as singlet oxygen). Suitable examples of an electron-rich olefin compound are listed in US Patent No. 5,709,994.

In addition to the olefin compound, the luminescent composition further includes a compound of a metal and one or more chelating agents (i.e., metal chelate). A metal of the metal chelate can be a rare earth metal or a group VIII metal, preferably selected from europium, terbium, dysprosium, samarium, osmium and ruthenium, and more preferably selected from europium.
the metal chelate can comprise a chelating agent selected from the following: 4'-(10-methyl-9-anthryl)-2,2':6'2"-bitripyridine-6,6"-dimethylamine]tetraacetic acid (MTTA), 2-(1',1',2',2',3',3'-heptafluoro-4',6'-hexanedione-6'-yl)-naphthalene
(NHA), 4,4'-bis(2",3",3"-heptafluoro-4",6"-hexanedione-6"-yl)-ortho-terphenyl (BHHT), 44'-bis(1",1",1",2",2",3",3" -heptafluoro-4",6"-hexanedione-6"-yl)-chlorosulfonyl-o-terphenyl (BHHCT), 4,7-biphenyl-1,10-phenanthroline (DPP), 1,1,1-trifluoroacetone (TTA), 3-naphthoyl-1,1,1-trifluoroacetone (NPPTA), naphthyl trifluorobutanedione (NTA), trioctyl phosphine oxide (TOPO), triphenyl phosphine oxide (TPPO), 3-benzoyl-1,1,1 -trifluoroacetone (BFTA), 2,2-dimethyl-4-perfluorobutyryl-3-butanone (fod), 2,2'-bipyridine (bpy), bipyridyl carboxylic acid, aza crown ether, aza cryptand, trioctyl phosphine oxide and their derivatives.

The luminescent compound can be a derivative of thioxene, and the metal chelate can be a europium chelate.

The substrate can be selected from strips, sheets, rods, tubes, apertures, microtiter plates, beads, particles and microspheres, and preferably selected from beads and microspheres.

The substrate can be magnetic or non-magnetic particles.

Materials of substrates for the receptor microspheres of different particle diameters can be the same or different.

A material of the substrate can be selected from natural, synthetic or modified naturally existing polymers, which include, but are not limited to: agarose, cellulose, nitrocellulose, cellulose acetate, polyvinyl chloride, polystyrene, polyethylene, polypropylene, poly(4-methylbutene), polyacrylamide, polymethacrylate, polyethylene terephthalate, nylon, polyvinyl butyrate or polyacrylate.

The substrate can be an aldehydized latex microsphere, and preferably an aldehydized polystyrene latex microsphere.

The substrate can be directly connected on a surface thereof with a biologically active substance, and the biologically active substance can be capable of specifically binding to a target molecule to be detected.

The substrate can be coated on a surface thereof with a coating layer. The coating layer is connected on a surface thereof with a biologically active substance, and the biologically active substance can be capable of specifically binding to a target molecule to be detected.

Examples of a binding partner for the biologically active substance-target molecule to be detected include: antigen-antibody, hormone-hormone receptor, nucleic acid duplex, IgG-protein A, polynucleotide pair such as DNA-DNA and DNA-RNA, and so on.

In some preferred embodiments of the present disclosure, the biologically active substance is an antibody and/or antigen. The antigen refers to a substance with immunogenicity, and the antibody refers to an immunoglobulin, produced by a body, which can recognize specific foreign substances.

A coating substance in the coating layer can be selected from polysaccharides, high molecular polymers or biological macromolecules, and preferably selected from polysaccharides.

The substrate can be coated on a surface thereof with a coating layer including at least two consecutive polysaccharide layers. A first polysaccharide layer and a second polysaccharide layer can be associated with each other spontaneously.

Each layer in the consecutive polysaccharide layers can be associated with any of layers in a previous polysaccharide layer spontaneously.

The polysaccharide can have a pendant functional group, and the functional group of the consecutive polysaccharide layers can have an electric charge that is opposite to an electric charge of the functional group of a previous polysaccharide layer.

The polysaccharide can have a pendant functional group, and the consecutive polysaccharide layers of the polysaccharide covalently can bind to a previous polysaccharide layer by a reaction between a functional group of the consecutive polysaccharide layers and a functional group of a previous polysaccharide layer.

The functional group of the consecutive polysaccharide layers canalternate between an amine functional group and an amine reactive functional group.

The amine reactive functional group can be aldehyde or carboxyl.

The first polysaccharide layer can be associated with a carrier spontaneously.

An outmost polysaccharide layer of the coating layer can have at least one pendant functional group.

The pendant functional group of the outmost polysaccharide layer of the coating layer can be selected from at least one of aldehyde, carboxyl, sulfhydryl, amino, hydroxyl and maleamido, and preferably selected from aldehyde and/or carboxyl.

The pendant functional group of the outmost polysaccharide layer of the coating layer can be directly or indirectly connected with the biologically active substance.

The polysaccharide can be selected from a carbohydrate having three or more unmodified or modified monosaccharide units; and preferably selected from glucan, starch, glycogen, inulin, fructan, mannan, agarose, galactan, carboxyglucan, and aminodextran; and the polysaccharide can be more preferably selected from glucan, starch, glycogen, and polyribosome.

Preferably, the substrates of the receptor microspheres of different particle diameters have the same particle diameter.

Preferably, the substrates of the receptor microspheres of different particle diameters have different particle diameters.

The active oxygen can be singlet oxygen.

Preferably, the microsphere composition includes two kinds of receptor microspheres of different particle diameters.

In some specific embodiments, a detection wavelength of the chemiluminescent signal is 450-680 nm, preferably 520-620 nm, more preferably 610-620 nm, and most preferably 615 nm. Correspondingly, a wavelength range of excitation light used for excitation is 640-680 nm, and preferably a wavelength of the excitation light is 660 nm.

Preferably, a difference between particle diameters of the two kinds of receptor microspheres of different particle diameters is not lower than 100 nm, preferably not lower than 150 nm, and more preferably not lower than 200 nm. In some specific embodiments, a difference between particle diameters of the two kinds of receptor microspheres of different particle diameters is not lower than 100nm, 130 nm, 150 nm, 170 nm, 190 nm, 200 nm, 220 nm, 240 nm or 250 nm.

Preferably, a particle diameter ratio of the two kinds of receptor microspheres of different particle diameters is selected from 1: (1.1-10), preferably selected from 1: (2-8), and more preferably selected from 1: (3-6). In some specific embodiments, a particle diameter ratio of the two kinds of receptor microspheres of different particle diameters is selected from 1:1.5, 1:2, 1:2.7, 1:3, 1:3.2, 1:3.75, 1:4, 1:5 or 1:6.

A used concentration of the microsphere composition can be 1 ug/mL-1000 ug/mL, preferably 10ug/mL-500ug/mL, and more preferably 10ug/mL-250ug/mL. The used concentration of the microsphere composition is determined by concentrations of different target molecules to be detected in the blood and properties of the target molecules to be detected.

Preferably, the microsphere composition includes at least three kinds of receptor microspheres of different particle diameters.

The invention also relates to a receptor reagent as claimed in claim 10, which comprises the microsphere composition according to the invention.

The invention also relates to a chemiluminescent detection kit as claimed in claim 11, which comprises the microsphere composition of the invention or the receptor reagent according to the invention. When used for detection, the kit has advantages of both a high sensitivity and a wide detection range.

The invention also relates to a chemiluminescent analysis method as claimed in claim 12, which detects whether a target molecule to be detected is present in a sample to be detected and/or detects a concentration of the target molecule to be detected in the sample to be detected by using the microsphere composition according to the invention, or the receptor reagent according to the invention, or the kit according to the invention. The method has advantages of both a high sensitivity and a wide detection range.

A chemiluminescent analyzer, which detects whether a target molecule to be detected can be present in a sample to be detected and/or can detect a concentration of the target molecule to be detected in the sample to be detected by using the microsphere composition according to the invention, or the receptor reagent according to the invention, or the kit, and/or the method according to the invention.

The chemiluminescent analyzer can comprise at least the following parts:
an incubation module, which is used to provide a suitable temperature environment for a chemiluminescent reaction between a sample to be detected and a microsphere composition, the microsphere composition including at least two kinds of receptor microspheres of different particle diameters;
a detection module, which is used to detect a chemiluminescent signal produced by a reaction between the receptor microspheres and active oxygen; and
a processor, which determines whether a target molecule to be detected is present in a sample to be detected and/or detects a concentration of the target molecule to be detected in the sample to be detected according to a circumstance about the chemiluminescent signal detected by the detection module.

The present disclosure has the following beneficial effects. The microsphere composition used for chemiluminescent analysis of the invention comprises at least two kinds of receptor microspheres of different particle diameters at the same time. Since the receptor microspheres having a small particle diameter can broaden a detection range and the receptor microspheres having a large particle diameter can improve the detection sensitivity, the microsphere composition of the inventionhas greatly improved performance compared with existing technologies, and has both an ultrahigh sensitivity and a very wide detection range. Besides, since the receptor microspheres having a small particle diameter have a fast reaction rate, a detection time length can be shortened when the microsphere composition of the inventionis used for detection, and thus a reaction speed is improved.

### Brief Description of the Drawings

The invention will be further described below with reference to the accompanying drawings.
Fig. 1 is a Gaussian distribution curve of an aldehyde polystyrene latex microsphere prepared in Example 3.
Fig. 2 is a Gaussian distribution curve of an aldehyde polystyrene latex microsphere embedded with a luminescent composition, prepared in Example 3.
Fig. 3 is a Gaussian distribution graph of an aldehyde polystyrene latex microsphere embedded with a luminescent composition and coated with glucan, prepared in Example 3.
Fig. 4 is a Gaussian distribution graph of a receptor microsphere having a mean particle diameter of about 250 nm prepared in Example 3.
Fig. 5 is a Gaussian distribution graph of a receptor microsphere having a particle diameter of about 110 nm prepared in Example 3.
Fig. 6 is a Nicomp distribution graph of the receptor microsphere having a particle diameter of about 110 nm prepared in Example 3.
Fig. 7 is a Gaussian distribution graph of a receptor microsphere having a particle diameter of about 350 nm prepared in Example 3.
Fig. 8 is a Nicomp distribution graph of the receptor microsphere having the particle diameter of about 350 nm prepared in Example 3.
Fig. 9 is a Gaussian distribution graph of the particle diameters distribution of the receptor microspheres mixture in Example 4.
Fig. 10 is a Nicomp distribution graph of the particle diameters distribution of the receptor microsphere mixture in Example 4.

### Detailed Description

In order to make the invention better understood, the invention will be described in detail below.

In a case that a numeral value range is provided, an upper limit and a lower limit of the range and each intervening value between any other specified or intervening values in the specified range is covered. The upper limits and lower limits of these smaller ranges may be individually included in the smaller ranges. In a case that the specified range includes one or two limits, the exclusion of either or both of the included limits is also included.

The term "active oxygen" refers herein to a general term of substances that are composed of oxygen, contain oxygen and are active in nature in a body or in the natural environment. It is mainly an excited-state oxygen molecule, including the one-electron reduction product of oxygen, i.e., superoxide anion (O₂·-), two-electron reduction product of oxygen, i.e., hydrogen peroxide (H₂O₂), three-electron reduction product, i.e., hydroxyl radical (·OH), nitric oxide, singlet oxygen (1O₂) and so on.

The term "receptor microsphere" refers to a nano-microsphere that can react with active oxygen to produce a detectable chemiluminescent signal, and it may also be called oxygen-receiving microsphere or luminescent microsphere. Preferably, the receptor microsphere may be a polymer particle filled with a luminescent composition formed by filling a substrate with functional groups, and the luminescent composition includes a chemiluminescent compound capable of reacting with active oxygen. The chemiluminescent compound can be subjected to a chemical reaction with active oxygen to so as form an unstable metastable intermediate, which can decompose and emit light at the same time or subsequently. Typical examples of these substances include, but are not limited to: enol ether, enamine, 9-alkylidene xanthan gum, 9-alkylidene-N-alkyl acridine, aryl etherene, dioxirene, thioxene, aryl imidazole or lucigenin.

The "chemiluminescent compound" is a compound which is called label, and can be used for a chemical reaction to cause luminescence, for example, by being converted into another compound formed in an electronically excited state. The electronically excited state may be a singlet state or a triplet state. The excited state can relax to the ground state, so that light is emitted directly, or it can return to the ground state by transferring excitation energy to a receptor of emission energy. During this process, an energy receptor microsphere transitions to an excited state and emits light.

The chemiluminescent compound may bind to a specific binding partner member that can directly or indirectly bind to a target molecule to be detected or a test component, and a concentration of the test component is affected by the presence of the target molecule to be detected. The term "can directly or indirectly bind to" means that a specified physical object can specifically bind to a physical object (directly), or the specified physical object can specifically bind to a specific binding pair member, or to a compound having two or more specific binding partners that can bind to other physical objects (indirectly).

The "specific binding pair member" is selected from the following: (1) a small molecule and a binding partner for the small molecule; and (2) a macromolecule and a binding partner for the macromolecule.

The active oxygen can be provided by a "donor microsphere", which is nano-microsphere capable of generating active oxygen in an excited state. Preferably, the donor microsphere can be coated on a substrate through functional groups to form a polymer particle filled with a photosensitive compound; and the donor microsphere can generate singlet oxygen under light excitation, and at this time, the donor microsphere can also be called oxygen supply microsphere or photosensitive microsphere. The donor microsphere can have hydrophilic aldehyde dextran on the surface, and the interior thereof is filled with a photosensitizer. The photosensitizer may be a photosensitizer known in the art, preferably a compound that is relatively light-stable and does not react effectively with singlet oxygen, non-limiting examples of which include compounds such as methylene blue, rose red, porphyrin, and phthalocyanine, and derivatives, of these compounds, with 1-50 atom substituents. The substituents are used to make these compounds more lipophilic or more hydrophilic, and/or serve as a linking group linked to the specific binding pair member. The surface of the donor microsphere can also be filled with other sensitizers, non-limiting examples of which are certain compounds that catalyze the conversion of hydrogen peroxide into singlet oxygen and water. Examples of some other donors include: 1,4-dicarboxyethyl-1,4-naphthalene endoperoxide, 9,10-diphenylanthracene-9,10-endoperoxide, and so on, and these compounds release active oxygen, such as singlet oxygen, by heating or by directly absorbing light.

The sensitizer is a compound that, when excited or induced to react, can cause a chemical reaction of another compound or substance. The sensitizer includes a photosensitizer, which can be induced by light irradiation to form an activated excited state. The sensitizer also includes a compound that can react chemically to produce a metastable reactive oxygen substance such as singlet oxygen.

The chemiluminescent compound is generally activated by the photosensitizer through irradiation performed to a medium containing the above-mentioned reactant. The medium must be irradiated with light having a certain wavelength and sufficient energy to convert the photosensitizer to an excited state, which further enable the photosensitizer to activate molecular oxygen into singlet oxygen. The excited state of the photosensitizer capable of exciting molecular oxygen is generally in the triplet state, which has energy about 20 Kcal/mol, usually at least 23 Kcal/mol, higher than energy of the photosensitizer in the ground state. Although a shorter wavelength such as 230-950 nm can be used, it is preferable to use light with a wavelength of approximately 450-950 nm to irradiate the medium. The light generated can be measured in any conventional way, such as photography, visual inspection, photometer, and so on, in order to determine its amount related to the analyte content in the medium. The photosensitizer is preferably a relatively non-polar compound to ensure solubility into a lipophilic member.

The "substrate" may be of any size, may be organic or inorganic, may be expandable or non-expandable, may be porous or non-porous, may have any density, but preferably have a density close to that of water and preferably is capable of floating in water. The "substrate" is formed by a transparent, partially transparent or opaque material. The substrate may have or may not have a charge, and when the substrate has a charge, the charge is preferably a negative charge. The substrate can be solid (such as polymers, metals, glass, organic and inorganic substances such as minerals, salts and diatoms), small oil droplets (such as hydrocarbons, fluorocarbons and siliceous fluids), vesicles (for example, synthetic ones, such as phospholipids, or natural ones, such as cells and cell organs). The substrate can be latex particles or other particles containing organic or inorganic polymers, lipid bilayers such as liposomes, phospholipid vesicles, small oil droplets, silicon particles, metal sols, cells and microcrystalline dyes. The substrate is usually multifunctional or capable of binding to a donor or receptor through specific or non-specific covalent or non-covalent interactions. Many functional groups are available or may be incorporated. Typical functional groups include carboxylic acid, acetaldehyde, amino, cyano, vinyl, hydroxyl, sulfhydryl and the like. A non-limiting example of the substrate is aldehyde polystyrene latex microsphere.

The photosensitizer and/or the chemiluminescent compound can be selected to be dissolved in, or non-covalently bind to the surface of the particle. In this case, the photosensitizer and the chemiluminescent compound are preferably hydrophobic to reduce their ability to dissociate from the particle, so that both the photosensitizer and the chemiluminescent compound can bind to the same particle.

The term "particle diameter" refers to a mean particle diameter of the receptor microsphere, which is measured by a conventional particle diameter analyzer. The "receptor microsphere" includes at least a substrate, a luminescent composition and a biologically active molecule, and preferably further includes a coating layer. The luminescent composition can be filled in the substrate and/or coated on the surface of the substrate. When the receptor microsphere does not include a coating layer, the biologically active substance is directly connected to the surface of the substrate. When the receptor microsphere includes a coating layer, the coating layer is coated on the surface of the substrate, and the outermost layer of the coating layer is connected with the biologically active substance.

The "mean particle diameter of the receptor microsphere" refers to the mean particle diameter of the receptor microsphere after being connected and/or coated with a corresponding substance. Particle diameters of the substrates in the receptor microspheres of different particle diameters can be the same or different, as long as the particle diameters of the finally formed receptor microspheres are different. The most preferred technical is that the particle diameters of the substrates in the receptor microspheres of different particle diameters are different.

The references to methods of treatment by therapy of surgery or in vivo diagnosis methods below are to be interpreted as references to compounds of use in those methods.

The term "sample to be detected" refers to a mixture containing or suspected of containing the target molecule to be detected. The sample to be detected that can be used includes body fluids, such as blood (which can be anticoagulated blood commonly seen in collected blood samples), plasma, serum, urine, semen, saliva, cell culture, tissue extracts, and so on. Other types of the sample to be detected include solvents, seawater, industrial water samples, food samples, environmental samples such as soil or water, plant materials, eukaryotic cells, bacteria, plasmids, viruses, fungi, and cells derived from prokaryotes. The sample to be detected can be diluted with a diluent as needed before use. For example, in order to avoid the HOOK effect, the sample to be detected can be diluted with a diluent before being detected on a detection instrument, and then is detected on the detection instrument.

The term "target molecule to be detected" refers to the substance in the sample to be detected during detection. One or more substances with specific binding affinity to the target molecule to be detected will be used to detect the target molecule. The target molecule to be detected can be a protein, peptide, antibody or a hapten that can bind to the antibody. The target molecule to be detected can be a nucleic acid or oligonucleotide that binds to a complementary nucleic acid or oligonucleotide. The target molecule to be detected can be any other substance that can form a specific binding pair member. Other typical examples of the target molecule to be detected include: drugs, such as steroids, hormones, proteins, glycoproteins, mucins, nucleoproteins, phosphoproteins, drugs of abuse, vitamins, antibacterial drugs, antifungal drugs, antiviral drugs, purines, anti-tumor agents, amphetamines, aza compounds, nucleic acids and prostaglandins, and metabolites of any of these drugs; pesticides and their metabolites; and receptors. Analytes also include cells, viruses, bacteria, and fungi.

The term "antibody" is used in the broadest sense. The antibody includes any alloantibodies, and antibody fragments that retain specific binding to an antigen. The antibody includes, but is not limited to, Fab, Fv, scFv, and Fd fragments, chimeric antibodies, humanized antibodies, single-chain antibodies, bispecific antibodies, and fusion proteins containing the antigen-binding portion of the antibody and non-antibody proteins. If necessary, the antibody can be further conjugated with other substances, for example, a specific binding pair member, such as biotin or streptavidin (one of biotin-streptavidin specific binding pair members).

The term "antigen" refers to a substance that can stimulate the body to produce an immune response, and can bind to an immune response product, i.e., antibodies, and sensitized lymphocytes in vivo and in vitro to produce an immune effect.

The term "bind" refers to the direct association between two molecules due to interactions, such as covalent interaction, electrostatic interaction, hydrophobic interaction, ionic bonding and/or hydrogen bonding , including but not limited to salt-bridge and water-bridge interactions.

The term "specific binding" refers to the mutual discrimination and selective binding reaction between two substances. From the perspective of the three-dimensional structure, it is the conformational correspondence between the corresponding reactants. The detection method of the specific binding reaction includes, but is not limited to: double antibody sandwich method, competition method, neutralization competition method, indirect method or capture method.

The "coefficient of variation (C.V) value of a particle diameter distribution" refers to the coefficient of variation of the particle diameter in Gaussian distribution in a detection result of a nanoparticle diameter analyzer. The calculation formula of the coefficient of variation is: C.V value = (standard deviation SD / mean value) × 100%.

The term "Nicomp distribution" refers to an algorithm distribution in the US PSS nanoparticle diameter analyzer NICOMP. Compared with the Gaussian single-peak algorithm, the Nicomp multi-peak algorithm has unique advantages for analysis of a liquid dispersion system with multiple components and a heterogeneous particle diameter distribution and stability analysis of a colloidal system.

The invention deals with the control of a particle diameter of a receptor microsphere in a microsphere composition so as to further control an amount of a biologically active substance (e.g., an antibody/antigen) on a surface of each receptor microsphere (specifically, the microsphere with a small particle diameter has a large specific surface area, and thus has a large amount of reporter molecules on the surface of the microsphere per unit mass, and the microsphere with a large particle diameter has a small specific surface area, and thus has a small amount of reporter molecules on the surface of the microsphere per unit mass), thereby improving a detection sensitivity and broadening a detection range. In addition, since the receptor microsphere with a small particle diameter has a relatively small diameter, an activation efficiency of singlet oxygen generated by a donor microsphere is improved according to the invention, and a light-emitting efficiency of the receptor microsphere is improved according to the invention.

In some preferred specific embodiments, a particle diameter of one kind of receptor microsphere in the microsphere composition is selected from 50 nm-300 nm, while a particle diameter of the other kind of receptor microsphere in the microsphere composition is selected from 200 nm-400 nm. For example, the particle diameter of one kind of receptor microsphere in the microsphere composition is selected from 50 nm, 80 nm, 110 nm, 140 nm, 170 nm, 200 nm or 300 nm, while the particle diameter of the other kind of receptor microsphere in the microsphere composition is selected from 200 nm, 250 nm, 300 nm, 350 nm or 400 nm. Preferably, the particle diameter of one kind of receptor microsphere in the microsphere composition is selected from 50 nm-200 nm, while the particle diameter of the other kind of receptor microsphere in the microsphere composition is selected from 200 nm-350 nm.

In some most preferred specific embodiments, the particle diameter of one kind of receptor microsphere in the microsphere composition is selected from 80 nm-150 nm, while the particle diameter of the other kind of receptor microsphere in the microsphere composition is selected from 220 nm-350 nm. The particle diameter of the receptor microspheres is such that a uniform and stable latex solution can be produced. Generally, the particle diameter of the receptor microsphere that can meet this requirement should be in the nanometer range. Therefore, an upper limit of the particle diameter of the receptor microsphere having a large particle diameter is such that a stable latex solution can be produced, and is generally selected to be about 300 nm. At the same time, it should be possible that coating and washing of the receptor microsphere are carried out under existing technical conditions to meet the production of reagents.

In some embodiments, compositions and chemical structures of the receptor microspheres of different particle diameters may be the same or different. For example, respective luminescent compositions and/or substrates of the receptor microspheres of different particle diameters may be the same or different, as long as the receptor microspheres can react with active oxygen to produce a detectable chemiluminescent signal.

The receptor reagent comprises the microsphere composition of the invention.

In some specific embodiments, a coefficient of variation (C.V) value of a particle diameter distribution of the microsphere composition in the receptor reagent may be 5%, 6%, 7%, 8%, 9%, 10%, 11%, 12%, 13%, 14%, 15%, 16%, 17%, 18%, 19%, 20%, 25% , 30%, 35% or 40%.

It should be noted that, the coefficient of variation (C.V) value of a particle diameter distribution of the microsphere composition refers to a coefficient of variation (C.V) value of a particle diameter distribution of the receptor microsphere after being coated with the desired substances.

### Examples

The invention is further detailed with reference to examples. These examples are illustrative only. Unless otherwise noted, raw materials or components used in the present disclosure are all available on the market or can be formed by conventional methods.

### Example 1 Preparation of a microsphere composition

(1) Preparation and characterization processes of aldehyde polystyrene latex microspheres
   1. A 100 ml three-necked flask was prepared, and 40 mmol of styrene, 5 mmol of acrolein, and 10 ml of water were added. After stirring was performed for 10 min, N₂ was introduced for 30 min.
   2. 0.11 g of ammonium persulfate and 0.2 g of sodium chloride were weighed and dissolved in 40 ml of water to prepare an aqueous solution. The aqueous solution was added to the reaction system in step 1, and introduction of N₂ continued for 30 min.
   3. The reaction system was heated to 70°C, and a reaction was performed for 15 hours.
   4. An obtained emulsion after the reaction was cooled to room temperature and was filtered with a suitable filter cloth. Then the emulsion was washed by centrifugal sedimentation with deionized water repeatedly until a conductivity of a centrifuged supernatant was close to that of the deionized water, and then an obtained product was diluted with water and stored as an emulsion.
   5. A particle diameter of latex microspheres measured by a nanoparticle diameter analyzer was 190.4 nm, C.V=5.1%; and a content of aldehyde in the latex microspheres measured by a conductivity titration method was 280 nmol/mg.
(2) Coating process and characterization process of a luminescent composition
   1. A 25 ml round bottom flask was prepared, and 0.1g of a thioxene derivative, 0.1g of europium (III) complex (MTTA-EU³⁺), and 10ml of 95% ethanol were added. An obtained mixture was magnetically stirred, and was heated to 70°C in water bath to obtain a complex solution.
   2. A 100 ml three-necked flask was prepared, and 10 ml of 95% ethanol, 10 ml of water, and 10 ml of aldehyde polystyrene latex microspheres obtained in step (1) with a concentration of 10% and a particle diameter of 190.4nm were added. An obtained mixture was magnetically stirred, and was heated to 70°C in water bath.
   3. The complex solution in step 1 was slowly added dropwise to the three-necked flask in step 2. After a reaction was performed at 70°C for 2 hours, stirring was stopped, and an obtained emulsion was cooled naturally.
   4. The above emulsion was centrifuged for 1 hour at 30000G. A supernatant was discarded after centrifugation, and resuspending was performed with 50% ethanol. After the above centrifugal washing was repeated three times, resuspending was performed by using a 50 mM CB buffer solution with pH=10 to make an obtained product have a final concentration of 20 mg/ml.
(3) Process of coupling to an antibody
   1. PCT antibody 1 was dialyzed to a 50 mM CB buffer solution with pH=10, and a measured concentration was 1 mg/ml.
   2. 0.5 ml of polystyrene latex microspheres with aldehyde obtained in step (2) and 0.5 ml of PCT antibody 1 obtained in step 1 were added into a 2 ml centrifuge tube and mixed uniformly, and then 100 µl of a 50mM CB buffer solution containing 10mg NaBH₄ per milliliter was added, and a reaction was performed at 2-8°C for 4 hours.
   3. After the reaction was finished, 0.5 ml of a 50mM CB buffer solution containing 100mg BSA per milliliter was added, and a reaction was performed at 2-8°C for 2 hours.
   4. After the reaction was finished, centrifugation was performed for 45 min at 30000G. A supernatant was discarded after centrifugation, and resuspending was performed with a 50 mM MES buffer solution. Centrifugal washing was repeated four times to obtain receptor microspheres coupled to the PCT antibody 1.
   5. A particle diameter of the receptor microspheres measured by the nanoparticle diameter analyzer was 210.4 nm, C.V=5.1%.

Receptor microspheres with a particle diameter of 50 nm, 80 nm, 110 nm, 140 nm, 170 nm, 250 nm, 300 nm, 350 nm, 400 nm were prepared respectively by using the above same method.

### Example 2 Measurement of a sensitivity and an upper limit of detection of when using a microsphere composition for detection

A sensitivity point is defined as: when a detection signal value of an analyte concentration of 2C0 is higher than twice the detection signal value of an analyte concentration of C0, i.e., RLU(2C0)>2RLU(C0), a sensitivity of a corresponding detection reagent is C0. An upper limit point of detection is defined as: a corresponding concentration obtained through back calculation by putting a detection signal at a concentration of 1000 ng/ml into a concentration-signal value curve .
(1) A cTnI antigen was diluted to a series of concentrations, i.e., 5 pg/ml, 10 pg/ml, 20 pg/ml, 30 pg/ml, 40 pg/ml, 50 pg/ml, 100 pg/ml, 1000 pg/ml, 5000 pg /ml, 10000 pg/ml, 50000 pg/ml, 1000ng/ml. Receptor microspheres coated with a cTnI monoclonal antibody 1 of different particle diameters (i.e., 50nm, 80nm, 110nm, 140nm, 170nm, 200nm, 250nm, 300nm, 350nm, 400nm) prepared by the same method as in Example 1 were diluted to 100ug/ml respectively. Then the above diluted acceptor microsphere and a cTnI monoclonal antibody 2 labeled with the same biotin (diluted to 2 ug/ml) and a general solution (a solution of donor microspheres) were used for a detection of the cTnI antigen at the above-mentioned series of concentrations, and a detection sensitivity and an upper limit of detection are shown in Table 1.
(2) A PCT antigen was diluted to a series of concentrations, i.e., to 20 pg/ml, 30 pg/ml, 40 pg/ml, 60 pg/ml, 80 pg/ml, 160 pg/ml, 500 pg/ml, 1000 pg/ml, 5000 pg/ml, 20000 pg/ml, 100000 pg/ml and 2000ng/ml. Receptor microspheres coated with a PCT monoclonal antibody 1 of different particle diameters (i.e., 50 nm, 80 nm, 110 nm, 140nm, 170 nm, 200 nm, 250 nm, 300 nm, 350 nm , 400 nm) prepared by the same method as in Example 1 were diluted to 100ug/ml respectively. Then the above diluted acceptor microsphere and a PCT monoclonal antibody 2 labeled with the same biotin (diluted to 2 ug/ml) and a general solution (a solution of donor microspheres) were used for detection of the PCT antigen at the above-mentioned series of concentrations, and a detection sensitivity and an upper limit of detection are shown in Table 1.

**Table 1**

| Particle diameter of receptor microspheres /nm | cTnI | | PCT | |
|---|---|---|---|---|
| | Sensitivity pg/ml | Upper limit of detection ng/ml | Sensitivity pg/ml | Upper limit of detection ng/ml |
| 50 | 50 | 148.67 | 160 | 685.97 |
| 80 | 50 | 144.19 | 160 | 694.76 |
| 110 | 50 | 142.05 | 80 | 634.11 |
| 140 | 30 | 122.06 | 60 | 465.67 |
| 170 | 20 | 94.69 | 60 | 387.01 |
| 200 | 20 | 88.74 | 40 | 346.43 |
| 250 | 10 | 73.56 | 40 | 272.06 |
| 300 | 10 | 61.11 | 30 | 232.54 |
| 350 | 5 | 44.05 | 30 | 217.74 |
| 400 | 5 | 32.75 | 30 | 204.37 |

Detection results for a cTnI project and detection results for a PCT project are shown in Table 1.
(1) Detection results for the cTnI project: When receptor microspheres having a particle diameter of 50 nm and receptor microspheres having a particle diameter of 80 nm are used for detection, the upper limits of detection are very high, but the sensitivity are relatively poor. When receptor microspheres having a particle diameter of 300 nm are used for detection, the sensitivity is best, but the upper limit of detection is relatively low. Microsphere compositions were formed by mixing the receptor microspheres having a particle diameter of 50 nm and the receptor microspheres having a particle diameter of 80 nm respectively with the receptor microspheres having a particle diameter of 300 nm, and sensitivities and upper limits of detection when the corresponding microsphere compositions were used for detection were detected, and results are shown in Table 2.
(2) Detection results for the PCT project: When receptor microspheres having a particle diameter of 110 nm are used for detection, the upper limit of detection is very high, but the sensitivity is relatively poor. When receptor microspheres having a particle diameter of 300 nm and receptor microspheres having a particle diameter of 350 nm are used for detection, the sensitivities are best, but the upper limits of detection are relatively low. Microsphere compositions were formed by mixing the receptor microspheres having a particle diameter of 110 nm with the receptor microspheres having a particle diameter of 300 nm and the receptor microspheres having a particle diameter of 350 nm respectively, and sensitivities and upper limits of detection when the corresponding microsphere compositions were used for detection were detected, and results are shown in Table 2.

**Table 2**

| Combination of particle diameters of receptor microspheres /nm | | | Sensitivity pg/ml | Upper limit of detection ng/ml |
|---|---|---|---|---|
| cTnI | 50 | 300 | 5 | 140.5 |
| | 80 | 300 | 5 | 145.5 |
| PCT | 110 | 300 | 30 | 601.43 |
| | 110 | 350 | 30 | 673.25 |

As can be seen from Table 2, when a microsphere composition formed by combining receptor microspheres with a small particle diameter with receptor microspheres with a large particle diameter was used for detection, it has both a high sensitivity and a high upper limit of detection (a wide detection range), exhibiting advantages of the receptor microspheres with a small particle diameter and the receptor microspheres with a large particle diameter. Compared with receptor microspheres of a single particle diameter, performance of a microsphere composition with receptor microspheres of two and more particle diameters is greatly improved.

### Example 3 Preparation of a microsphere composition

### (I) Preparation of receptor microspheres coupled to an antibody with a mean particle diameter of about 250 nm

### 1.1 Preparation and characterization processes of aldehyde polystyrene latex microspheres

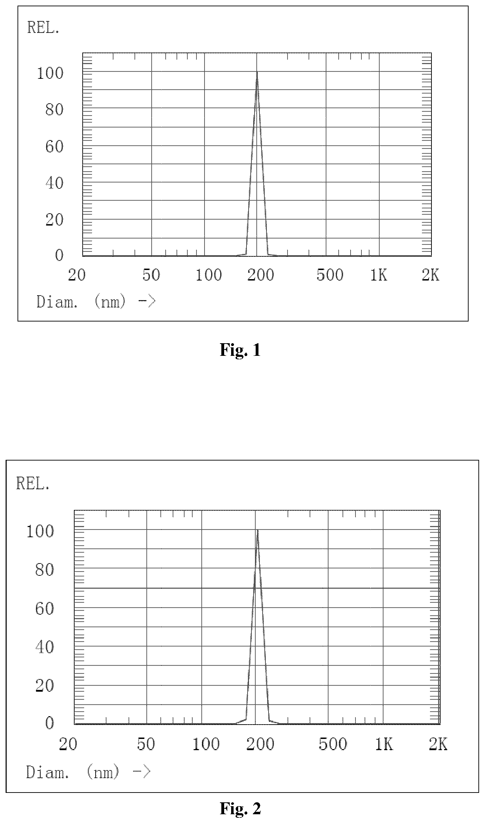

1). A 100 ml three-necked flask was prepared, and 40 mmol of styrene, 5 mmol of acrolein, and 10 ml of water were added. After stirring was performed for 10 min, N₂ was introduced for 30 min.
2). 0.11 g of ammonium persulfate and 0.2 g of sodium chloride were weighed and dissolved in 40 ml of water to prepare an aqueous solution. The aqueous solution was added to the reaction system in step 1, and introduction of N₂ continued for 30 min.
   3. The reaction system was heated to 70°C, and a reaction was performed for 15 hours.
   4. An obtained emulsion after the reaction was cooled to room temperature and was filtered with a suitable filter cloth. Then the emulsion was washed by centrifugal sedimentation with deionized water repeatedly until a conductivity of a supernatant was close to that of the deionized water, and then an obtained product was diluted with water and stored as an emulsion.
   5. A mean particle diameter of Gaussian distribution for particle diameters of latex microspheres at this time measured by a nanoparticle diameter analyzer was 202.2 nm, a coefficient of variation (C.V)=4.6%, and a Gaussian distribution curve is shown in Fig. 1. A content of aldehyde in the latex microspheres measured by a conductivity titration method was 280 nmol/mg.

### (2) Embedding process and characterization process of a luminescent composition

1). A 25 ml round bottom flask was prepared, and 0.1g of a thioxene derivative, 0.1g of europium (III) complex (MTTA-EU³⁺), and 10ml of 95% ethanol were added. An obtained mixture was magnetically stirred, and was heated to 70°C in water bath to obtain a complex solution.
2). A 100 ml three-necked flask was prepared, and 10 ml of 95% ethanol, 10 ml of water, and 10 ml of aldehyde polystyrene latex microspheres with a concentration of 10% obtained in process 1.1 were added. An obtained mixture was magnetically stirred, and was heated to 70°C in water bath.
3). The complex solution obtained in step 1) was slowly added dropwise to the three-necked flask in step 2). After a reaction was performed at 70°C for 2 hours, stirring was stopped, and an obtained emulsion was cooled naturally.
4). The above emulsion was centrifuged for 1 hour at 30000G. A supernatant was discarded after centrifugation, and aldehyde polystyrene microspheres embedded with a luminescent composition were obtained.
5). A mean particle diameter of Gaussian distribution for particle diameters of microspheres at this time measured by a nanoparticle diameter analyzer was 204.9 nm, a coefficient of variation (C.V)=5.00% (as shown in Fig. 2).

### 1.3 Coating glucan on a surface of receptor microspheres

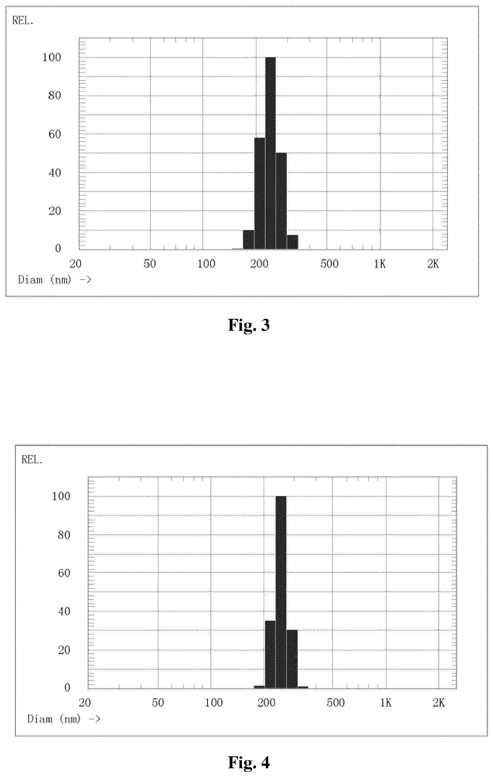

1) 50 mg of solid aminodextran was taken and placed in a 20 mL round bottom flask, and 5 mL of a 50 mM carbonate buffer solution with pH=10 was added. Then, stirring was performed at 30°C with the exclusion of light for dissolution, and an aminodextran solution was obtained.
2) 100 mg of the prepared aldehyde polystyrene microspheres embedded with a luminescent composition were taken and added to an aminodextran solution. An obtained mixture was stirred for 2 hours to obtained a reaction solution .
3) 10 mg of sodium borohydride was dissolved in 0.5 mL of a 50 mM carbonate buffer solution with pH=10, and an obtained solution was added dropwise to the above reaction solution for a reaction at 30°C with the exclusion of light overnight.
4) A mixture containing microspheres after the reaction was centrifuged at 30000G, and a supernatant was discarded after centrifugation, and then a 50 mM carbonate buffer solution with pH=10 was added, and the microspheres were dispersed by ultrasound. After the above centrifugal washing was repeated three times, the 50 mM carbonate buffer solution with pH=10 was added to providing a fixed volume, so that a final concentration of the microspheres was 20 mg/ml.
5) 100 mg of solid aldehyde dextran was taken and placed in a 20 mL round bottom flask, and 5 mL of the 50 mM carbonate buffer solution with pH=10 was added. Then, stirring was performed at 30°C with the exclusion of light for dissolution, and an aldehyde dextran solution was obtained.
6) The above microspheres obtained in step 4) were added into the aldehyde dextran solution. An obtained mixture was stirred for 2 hours to obtain a reaction solution.
7) 15 mg of sodium borohydride was dissolved in 0.5 mL of the 50 mM carbonate buffer solution with pH=10, and an obtained solution was added dropwise to the above reaction solution for a reaction at 30°C with the exclusion of light overnight.
8) A mixture containing microspheres after the reaction was centrifuged at 30000G, and a supernatant was discarded after centrifugation, and then a 50 mM carbonate buffer solution with pH=10 was added, and the microspheres were dispersed by ultrasound. After the above centrifugal washing was repeated three times, the 50 mM carbonate buffer solution with pH=10 was added to providing a fixed volume, so that a final concentration of the microspheres was 20 mg/ml.
9) A mean particle diameter of Gaussian distribution for particle diameters of microspheres at this time measured by a nanoparticle diameter analyzer was 241.6 nm, a coefficient of variation (C.V)=12.90% (as shown in Fig. 3).

### 1.4 Process of coupling to an antibody

1. A paired antibody I was dialyzed to a 50 mM CB buffer solution with pH=10, and a measured concentration is 1 mg/ml.
2. 0.5 ml of receptor microspheres obtained in process 1.3 and 0.5 ml of the paired antibody I obtained in step 1) were added into a 2 ml centrifuge tube and mixed uniformly, and then 100 µl of a 50mM CB buffer solution containing 10mg NaBH₄ per milliliter was added, and a reaction was performed at 2-8°C for 4 hours.
3. After the reaction was finished, 0.5 ml of a 50mM CB buffer solution containing 100mg BSA per milliliter was added, and a reaction was performed at 2-8°C for 2 hours.
4. After the reaction was finished, centrifugation was performed for 45 min at 30000G. A supernatant was discarded after centrifugation, and resuspending was performed with a 50 mM MES buffer solution. Centrifugal washing was repeated four times, and a final concentration of 100 µg/ml was obtained by dilution, thereby obtaining a solution of receptor microspheres coupled to the antibody 1.
5. A mean particle diameter value of Gaussian distribution for particle diameters of microspheres at this time measured by a nanoparticle diameter analyzer was 253.5 nm, a coefficient of variation (C.V value)=9.60% (as shown in Fig. 4).

### (II) Preparation of receptor microspheres coupled to an antibody with a mean particle diameter of about 110 nm

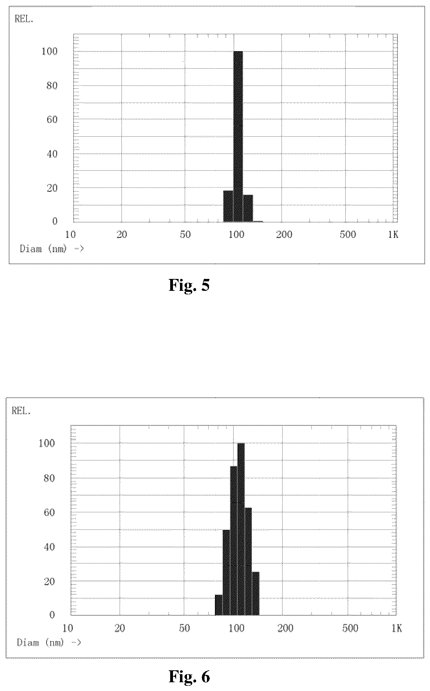

The preparation process is the same as the preparation process of receptor microspheres with a mean particle diameter of about 250 nm in (I). A mean particle diameter value of Gaussian distribution (as shown in Fig. 5) for particle diameters of the receptor microspheres measured by a nanoparticle diameter analyzer was 107.1 nm, a coefficient of variation (C.V)=7.60% . A Nicomp distribution for particle diameters of the acceptor microspheres is unimodal (as shown in Fig. 6).

### (III) Preparation of receptor microspheres coupled to a PCT antibody with a mean particle diameter of about 350 nm

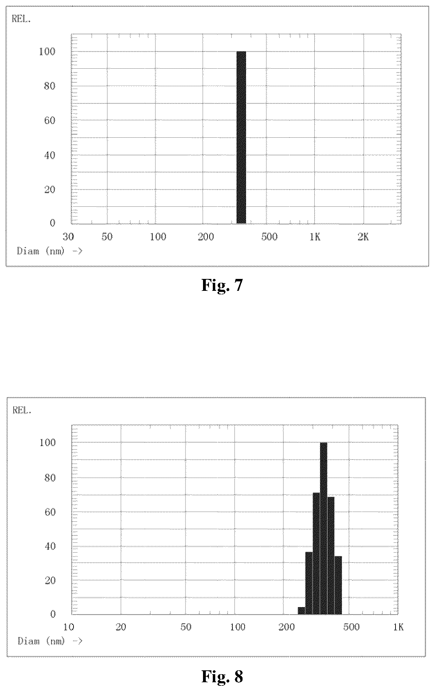

The preparation process is the same as the preparation process of receptor microspheres with a mean particle diameter of about 250 nm in (I). A mean particle diameter value of Gaussian distribution (as shown in Fig. 7) for particle diameters of the receptor microspheres measured by a nanoparticle diameter analyzer was 347.5 nm, a coefficient of variation (C.V)=3.9% . A Nicomp for particle diameters of the acceptor microspheres distribution is unimodal (as shown in Fig. 8).

### Example 4 Measurement of a sensitivity and an upper limit of detection of a kit

A sensitivity point is defined as: when a detection signal value of an analyte concentration of Cx is higher than twice the detection signal value of an analyte concentration of C0, i.e., RLU(Cx)>2RLU(C0), a sensitivity of a corresponding detection reagent is Cx. An upper limit point of detection is defined as an upper limit of scope determined in documents of series 6 of evaluation protocols (EP) of the National Committee for Clinical Laboratory Standards (NCCLS).
(1) A PCT antigen was diluted to a series of concentrations, i.e., 20 pg/ml, 30 pg/ml, 40 pg/ml, 50 pg/ml, 60 pg/ml, 80 pg/ml, 160 pg/ml, 500 pg/ml, 1000 pg/ml, 5000 pg/ml, 20000 pg /ml, 50000pg/ml, 100000pg/ml and 200000pg/ml. Receptor reagents (with a concentration of receptor microspheres of 100 ug/ml) respectively containing receptor microspheres with different mean particle diameters (110 nm, 250 nm and 350 nm) and coupled to the PCT antibody I prepared in Example 3 , and a PCT monoclonal antibody 2 labeled with the same biotin (diluted to 2 ug/ml) and a general solution (a reagent containing donor microspheres) were used for detection of the PCT antigen at the above-mentioned series of concentrations, and a detection sensitivity and an upper limit of detection by using a photo-excited chemiluminescent analysis system developed by Shanghai Beyond Biotech Co., Ltd are shown in Table 3.

**Table 3**

| Mean particle diameter of receptor microspheres /nm | PCT | |
|---|---|---|
| | Sensitivity pg/ml | Upper limit of detection ng/ml |
| 110 | 80 | 534.11 |
| 250 | 25 | 343.92 |
| 350 | 20 | 237.74 |

As can be seen from Table 3, when receptor microspheres having a particle diameter of 110 nm were used for detection, an upper limit of detection is very high, but a sensitivity is relatively poor; and when receptor microspheres having a particle diameter of 350 nm were used for detection, a sensitivity is best, but an upper limit of detection is relatively low.

(2) After mixing a solution of receptor microspheres with a mean particle diameter of 110 nm and coupled to the PCT antibody I and a solution of receptor microspheres with a mean particle diameter of 350 nm and coupled to the PCT antibody I, a new receptor reagent is obtained. In the new receptor reagent, measurement results of particle diameters of the receptor microspheres are as follows:

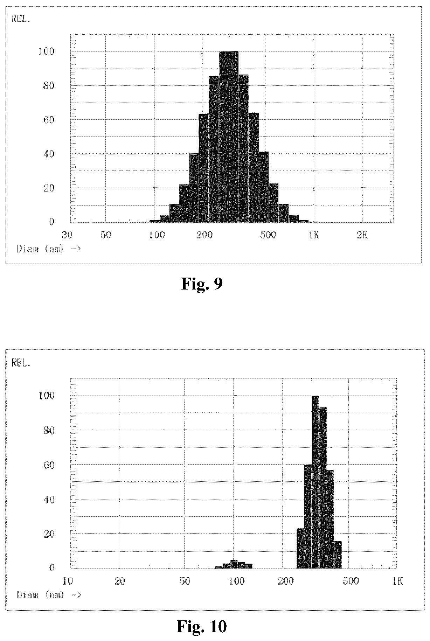

A mean particle diameter value of Gaussian distribution for particle diameters of the receptor microspheres was 317.7 nm, and a coefficient of variation of a particle diameter distribution (C.V value) =37.2% (as shown in Fig. 9).

A Nicomp distribution for particle diameters of the acceptor microspheres was bimodal. #1: a mean particle diameter was 103.1 nm, and a coefficient of variation (C.V value)=11.8%; and #2: a mean particle diameter was 328.8 nm, and a coefficient of variation of a particle diameter distribution(C.V value) =13.0% (as shown in Fig. 10).

Then, the above new receptor reagent, a PCT monoclonal antibody 2 labeled with a biotin (diluted to 2 ug/ml), and a general solution (a reagent containing donor microspheres) were used for detection of the PCT antigen at the above-mentioned series of concentrations, and a detection sensitivity and an upper limit of detection by using a photo-excited chemiluminescent analysis system developed by Shanghai Beyond Biotech Co., Ltd are shown in Table 4.

**Table 4**

| Combination of particle diameters of receptor microspheres /nm | | | Sensitivity pg/ml | Upper limit of detection ng/ml |
|---|---|---|---|---|
| PCT | 110 | 350 | 30 | 399.87 |

As can be seen from Table 4, by appropriately increasing heterogeneity of the particle diameter of the receptor microspheres, detection performance of the kit containing the receptor microspheres was significantly improved.

It should be noted that the above-mentioned embodiments are only used to explain the present disclosure and do not constitute any limitation to the present disclosure. The present disclosure has been described with reference to typical embodiments, but it should be understood that words used herein are descriptive and explanatory words, rather than restrictive words. The present disclosure may be modified within the scope of the claims of the present disclosure as stipulated, and the present disclosure may be revised without departing from the scope and spirit of the present disclosure. Although the present disclosure described relates to specific methods, materials and embodiments, it does not mean that the present disclosure is limited to specific examples disclosed herein. On the contrary, the present disclosure can be extended to all other methods and applications with the same functions.

## Claims

1. A microsphere composition for chemiluminescent analysis, which comprises at least two kinds of receptor microspheres of different particle diameters, and the receptor microspheres are capable of reacting with active oxygen to produce a detectable chemiluminescent signal.

2. The microsphere composition according to claim 1, wherein the receptor microsphere comprises two portions, i.e., a luminescent composition and a substrate, wherein the luminescent composition is filled in the substrate and/or is coated on a surface of the substrate.

3. The microsphere composition according to claim 2, wherein the luminescent composition is capable of reacting with active oxygen to produce a detectable chemiluminescent signal, and comprises a chemiluminescent compound and a metal chelate.

4. The microsphere composition according to any of claims 1 to 3, wherein the substrates of the receptor microspheres of different particle diameters have the same particle diameter.

5. The microsphere composition according to any of claims 1 to 3, wherein the substrates of the receptor microspheres of different particle diameters have different particle diameters.

6. The microsphere composition according to any of claims 1 to 5, wherein the microsphere composition comprises two kinds of receptor microspheres of different particle diameters.

7. The microsphere composition according to claim 6, wherein a difference between particle diameters of the two kinds of receptor microspheres of different particle diameters is not lower than 50 nm, preferably not lower than 100 nm, and more preferably not lower than 150 nm.

8. The microsphere composition according to claim 6 or 7, wherein a particle diameter ratio of the two kinds of receptor microspheres of different particle diameters is selected from 1: (1.1-10), preferably selected from 1: (2-8), and more preferably selected from 1: (3-6).

9. The microsphere composition according to any of claims 1-5, wherein the microsphere composition comprises at least three kinds of receptor microspheres of different particle diameters.

10. A receptor reagent, which comprises the microsphere composition according to any of claims 1-9.

11. A chemiluminescent detection kit, which comprises the microsphere composition according to any of claims 1-9 or the receptor reagent according to claim 10.

12. A chemiluminescent analysis method, which detects whether a target molecule to be detected is present in a sample to be detected and/or detects a concentration of the target molecule to be detected in the sample to be detected by using the microsphere composition according to any of claims 1-9,or the receptor reagent according to claim 10, or the kit according to claim 11.

## Patentansprüche

1. Mikrokugelzusammensetzung für die Chemilumineszenz-Analyse, wobei sie mindestens zwei Arten von Rezeptor-Mikrokugeln mit unterschiedlichen Partikeldurchmessern umfasst und die Rezeptor-Mikrokugeln dazu befähigt sind, mit aktivem Sauerstoff zu reagieren, um ein detektierbares Chemilumineszenzsignal zu erzeugen.

2. Mikrokugelzusammensetzung gemäß Anspruch 1, wobei die Rezeptor-Mikrokugel zwei Teilbereiche umfasst, nämlich eine lumineszente Zusammensetzung und ein Substrat, wobei die lumineszente Zusammensetzung in das Substrat gefüllt wird und/oder als Schicht auf eine Oberfläche des Substrats aufgebracht wird.

3. Mikrokugelzusammensetzung gemäß Anspruch 2, wobei die lumineszente Zusammensetzung dazu befähigt ist, mit aktivem Sauerstoff zu reagieren, um ein detektierbares Chemilumineszenzsignal zu erzeugen, wobei sie eine chemilumineszente Verbindung und ein Metallchelat umfasst.

4. Mikrokugelzusammensetzung gemäß einem beliebigen der Ansprüche 1 bis 3, wobei die Substrate der Rezeptor-Mikrokugeln mit verschiedenen Partikeldurchmessern denselben Partikeldurchmesser haben.

5. Mikrokugelzusammensetzung gemäß einem beliebigen der Ansprüche 1 bis 3, wobei die Substrate der Rezeptor-Mikrokugeln mit verschiedenen Partikeldurchmessern unterschiedliche Partikeldurchmesser haben.

6. Mikrokugelzusammensetzung gemäß einem beliebigen der Ansprüche 1 bis 5, wobei die Mikrokugelzusammensetzung zwei Arten von Rezeptor-Mikrokugeln mit unterschiedlichen Partikeldurchmessern umfasst.

7. Mikrokugelzusammensetzung gemäß Anspruch 6, wobei der Unterschied zwischen den Partikeldurchmessern der beiden Arten von Rezeptor-Mikrokugeln mit unterschiedlichen Partikeldurchmessern mindestens 50 nm beträgt, vorzugsweise mindestens 100 nm beträgt und insbesondere mindestens 150 nm beträgt.

8. Mikrokugelzusammensetzung gemäß Anspruch 6 oder 7, wobei das Verhältnis der Partikeldurchmessern der beiden Arten von Rezeptor-Mikrokugeln mit unterschiedlichen Partikeldurchmessern derart gewählt ist, dass es 1: (1,1 bis 10) beträgt, vorzugsweise derart gewählt ist, dass es 1: (2 bis 8) beträgt, und insbesondere derart gewählt ist, dass es 1: (3 bis 6) beträgt.

9. Mikrokugelzusammensetzung gemäß einem beliebigen der Ansprüche 1 bis 5, wobei die Mikrokugelzusammensetzung mindestens drei Arten von Rezeptor-Mikrokugeln mit unterschiedlichen Partikeldurchmessern umfasst.

10. Rezeptorreagenz, welches die Mikrokugelzusammensetzung gemäß einem beliebigen der Ansprüche 1 bis 9 umfasst.

11. Chemilumineszenz-Detektionskit, welches die Mikrokugelzusammensetzung gemäß einem beliebigen der Ansprüche 1 bis 9 oder das Rezeptorreagenz gemäß Anspruch 10 umfasst.

12. Chemilumineszenz-Analyseverfahren, welches entweder nachweist, ob ein zu detektierendes Zielmolekül in einer zu prüfenden Probe vorliegt, und/oder eine Konzentration des zu detektierenden Zielmoleküls in der zu prüfenden Probe detektiert, wobei zu diesem Zwecke die Mikrokugelzusammensetzung gemäß einem beliebigen der Ansprüche 1 bis 9 oder das Rezeptorreagenz gemäß Anspruch 10 oder das Kit gemäß Anspruch 11 zur Anwendung gebracht wird.

## Revendications

1. Composition de microsphères pour analyse par chimiluminescence, qui comprend au moins deux types de microsphères réceptrices avec des diamètre de particule différents, et les microsphères réceptrices sont capables de réagir avec l'oxygène actif pour produire un signal de chimiluminescence détectable.

2. Composition de microsphères selon la revendication 1, dans laquelle les microsphères réceptrices comprennent deux parties, à savoir une composition luminescente et un substrat, dans laquelle la composition luminescente est versée dans le substrat et/ou est appliquée en revêtement sur une surface du substrat.

3. Composition de microsphères selon la revendication 2, dans laquelle la composition luminescente est capable de réagir avec l'oxygène actif pour produire un signal de chimiluminescence détectable, et comprend un composé chimiluminescent et un chélate métallique.

4. Composition de microsphères selon l'une quelconque des revendications 1 à 3, dans laquelle les substrats des microsphères réceptrices avec des diamètre de particule différents présentent le même diamètre de particule.

5. Composition de microsphères selon l'une quelconque des revendications 1 à 3, dans laquelle les substrats des microsphères réceptrices avec des diamètres de particule différents présentent des diamètres de particule différents.

6. Composition de microsphères selon l'une quelconque des revendications 1 à 5, dans laquelle la composition de microsphères comprend deux types de microsphères réceptrices avec des diamètres de particule différents.

7. Composition de microsphères selon la revendication 6, dans laquelle une différence entre les diamètres de particule des deux types de microsphères réceptrices avec des diamètres de particule différents n'est pas inférieure à 50 nm, de préférence pas inférieur à 100 nm, et de manière plus préférée pas inférieur à 150 nm.

8. Composition de microsphères selon la revendication 6 ou 7, dans laquelle un rapport de diamètre de particule des deux types de microsphères réceptrices avec des diamètres de particule différents est choisi parmi 1: (1.1 à 10), de préférence choisi parmi 1: (2 à 8), et plus préférentiellement choisi parmi 1: (3 à 6).

9. Composition de microsphères selon l'une quelconque des revendications 1 à 5, dans laquelle la composition de microsphères comprend au moins trois types de microsphères réceptrices avec des diamètres de particule différents.

10. Réactif récepteur, qui comprend la composition de microsphères selon l'une quelconque des revendications 1 à 9.

11. Kit de détection par chimiluminescence, qui comprend la composition de microsphères selon l'une quelconque des revendications 1 à 9 ou le réactif récepteur selon la revendication 10.

12. Procédé d'analyse par chimiluminescence, qui détecte si une molécule cible devant faire l'objet d'une détection est présente dans un échantillon devant faire l'objet d'une et/ou détecte une concentration de la molécule cible devant faire l'objet d'une détection dans l'échantillon devant faire l'objet d'une détection en utilisant la composition de microsphères selon l'une quelconque des revendications 1 à 9, ou le réactif récepteur selon la revendication 10, ou le kit selon la revendication 11.
